# EUROPÄISCHE PATENTSCHRIFT

(11) **EP 2 305 346 B1**
(45) Veröffentlichungstag und Bekanntmachung des Hinweises auf die Patenterteilung: **13.04.2016**
(21) Anmeldenummer: 10011480.0
(22) Anmeldetag: 29.09.2010
(51) Int. Cl.: A61N 2/02

(54) **Vorrichtung zur Behandlung von Übergewicht**
Device for treating obesity
Dispositif de traitement d'une surcharge pondérale

(30) Priorität: 01.10.2009 DE 102009043728
(43) Veröffentlichungstag der Anmeldung: 06.04.2011
(73) Patentinhaber: Hesse, Albert, 57482 Wenden (DE)
(72) Erfinder: Hesse, Albert, 57482 Wenden (DE)
(74) Vertreter: von Renesse, Dorothea

(56) Entgegenhaltungen:
- EP-A1- 1 040 847
- WO-A1-97/46244
- DE-U1- 8 909 478
- US-A1- 2006 047 325
- US-A1- 2007 250 133

## Beschreibung

Die Erfindung betrifft eine Vorrichtung zur Behandlung von Übergewicht gemäß Anspruch 1.

Übergewicht (Adipositas, Fettleibigkeit, Fettsucht) ist eine typische Begleiterscheinung der Wohlstandsgesellschaft. Der Begriff "Übergewicht" bezeichnet eine über das Normalmaß hinausgehende Erhöhung des Körpergewichts und wird durch Indizes wie dem Broca-Index oder dem Body-Mass-Index (BMI) berechnet. Nach WHO Definition liegt Übergewicht bei einem BMI von mindestens 30 kg/m² vor.

Übergewicht ist demnach zumindest in den westlichen Industrieländern eine Volkskrankheit. Etwa jeder dritte erwachsene Bundesbürger ist übergewichtig. Im jüngeren Lebensalter sind eher Männer betroffen, ab dem vierzigsten Lebensjahr überwiegt der Anteil der Frauen. In den letzten Jahren wurde festgestellt, dass es immer mehr übergewichtige Kinder gibt. Diese Entwicklung ist insofern gefährlich, als übergewichtige Kinder ein erhöhtes Risiko haben, im Erwachsenenalter ernährungsabhängige Erkrankungen wie Bluthochdruck, Diabetes oder eine koronare Herzkrankheit zu entwickeln.

Die negativen gesundheitlichen Folgen des Übergewichts sind vielfältig und betreffen nahezu jedes Organ. Insbesondere sind das Herz und die Lunge belastet.

Bei Übergewichtigen muss das Herz ständig Mehrarbeit leisten, um das Fettgewebe ausreichend mit Blut zu versorgen. Außerdem wird im Körper vermehrt Wasser und Natrium zurückgehalten. Dies begünstigt die Entstehung von Bluthochdruck. Auch dies belastet das Herz. Die Folgen können Angina pectoris, Herzinfarkt oder akute oder chronische Herzinsuffizienz sein. Auch die Lunge muss, um den erhöhten Sauerstoffbedarf decken zu können, verstärkt arbeiten. Oft reicht aber die Lungenkapazität nicht aus, so dass sich ein chronischer Sauerstoffmangel entwickelt. Verstärkt wird dies noch durch Störungen der Atmung während des Schlafes (Schlaf-Apnoe-Syndrom). Die Betroffenen klagen über Schläfrigkeit während des Tages, lautes Schnarchen und ruhelosen Schlaf. Diese Beschwerden bilden sich nach Gewichtsreduktion meistens vollständig zurück.

Übergewicht ist der wichtigste Risikofaktor für die Entstehung des Diabetes mellitus Typ 2.

Besonders häufig sind Fettstoffwechselstörungen, die mit einer Erhöhung des für das Herz-Kreislaufsystem gefährlichen LDL-Cholesterins und der Triglyceride einhergehen. Außerdem drohen Leberverfettung, Gicht und Gallensteine.

Übergewicht führt zu einer Überlastung der Gelenke, besonders im unteren Wirbelsäulenbereich, in den Hüftgelenken sowie Knie- und Sprunggelenken. Dies beschleunigt den Verschleiß und führt zu chronischen Schmerzen, denen oft nur noch durch dauerhafte Einnahme von Schmerzmitteln oder einer Operation beizukommen ist.

Übergewicht führt jedoch nicht nur zu körperlichen Beeinträchtigungen, sondern kann auch seelische Folgen haben. So kann Übergewicht zu mangelndem Selbstbewusstsein, Isolation oder sogar Depressionen führen, die in einem Teufelskreis zu "Heißhungerattacken" und weiterer Gewichtszunahme führen.

Übergewicht kann nur entstehen, wenn die tägliche Kalorienaufnahme langfristig den Energieverbrauch des Körpers übersteigt (sog. positive Energiebilanz). Man geht heute davon aus, dass darüber hinaus bei der Entstehung von Übergewicht verschiedene Faktoren eine Rolle spielen. Neben Störungen im Essverhalten und mangelnder Bewegung spielt auch die Stoffwechselaktivität eine Rolle.

Zur Behandlung von Übergewicht existiert eine Vielzahl von Ansätzen, die im Wesentlichen auf den drei Säulen Ernährungs-, Bewegungs- und Verhaltenstherapie beruhen. Sie führen aber nur bei einem kleinen Teil der übergewichtigen Personen zu einer dauerhaften Reduzierung des Körpergewichtes. Dies beruht unter anderem darauf, dass eine dauerhaft erfolgreiche Therapie meist eine erhebliche Änderung zumindest einiger Lebens- und Eßgewohnheiten bei den Patienten voraussetzt. Daher brechen viele Patienten die Therapie vorzeitig ab, oder fallen nach Abschluss der Therapie wieder in ihre alten Lebensgewohnheiten zurück. Dies kann dann mit einer noch stärkeren Gewichtszunahme verbunden sein (sog. Jo-Jo-Effekt).

In besonders schweren Fällen der Adipositas müssen ergänzend zur Umstellung von Lebens- und Essgewohnheiten medikamentöse oder chirurgische Maßnahmen ergriffen werden. Diese sind für die Patienten oft zusätzlich belastend.

Über alle Therapieansätze hinweg ist daher die Therapietreue (engl. "compliance") der unter Übergewicht leidenden Patienten eher gering.

Aus der DE 100 62 050 A1 ist ein Verfahren zur Behandlung von Cellulitis bekannt, nach dem ein zeitlich variables Magnetfeld auf die Oberfläche des zu behandelnden Körperteils ausgeübt wird. Es wird auch angeregt, dieses Verfahren zur Behandlung von Fettleibigkeit einzusetzen. Es bleibt dem Fachmann nach dem Studium dieser Druckschrift aber unklar, ob und ggf. mit welchen Anpassungen eine Magnetfeldtherapie im Bereich der Fettleibigkeit überhaupt erfolgreich sein kann.

Die EP 1 040 847 A1 lehrt eine Vorrichtung gemäß dem Oberbegriff des Anspruchs 1. Der Erfindung liegt daher das Problem zugrunde, eine Methode bzw. eine Vorrichtung zur Behandlung von Übergewicht, insbesondere beim Menschen bereitzustellen, die eine möglichst hohe Therapietreue der Patienten erreichen kann. Wünschenswerterweise sollte die Methode/Vorrichtung zu einer dauerhaften Reduzierung des Körpergewichtes führen können, einfach in der Anwendung, bequem und/oder nebenwirkungsarm sein.

Die Aufgabe wird gelöst durch die Verwendung einer Vorrichtung mit einem ein die Schilddrüse aktivierendes Magnetfeld erzeugenden Modul zur Behandlung des Übergewichts bei Adipositas-Patienten, bzw. durch eine Vorrichtung zur Behandlung von Übergewicht bei einem Säuger, die ein Generatormodul zur Erzeugung eines Magnetfeldes und eine Steuereinheit umfasst und körpernah am Säuger befestigbar ist. Das Generatormodul erzeugt ein pulsierendes Magnetfeld, vorzugsweise mit einer Frequenz von 3 bis 60 Hz, bevorzugt mit einer Frequenz von 7 bis 20 Hz, insbesondere bevorzugt einer Frequenz von 14 Hz.

Das Magnetfeld der Vorrichtung erhöht die Schilddrüsentätigkeit des Patienten und führt damit mittelbar zu einer Gewichtsreduktion des Patienten. Der Kern der Erfindung besteht demnach darin, eine an sich bekannte Magnetfeldanwendung zu Zwecken der Gewichtsreduktion bei Adipositas-Patienten einzusetzen.

Vorzugsweise erfolgt erfindungsgemäß die Steuerung des Pulsierens des Magnetfelds elektronisch (nicht-mechanisch), also beispielsweise durch einen Schaltkreis. Dies hat gegenüber einer mechanischen Steuerung, wie etwa einem Rotieren eines Permanentmagneten, Vorteile, nämlich z.B. eine geringere Geräuschbelästigung, einen geringeren Energieverbrauch und/oder eine erhöhte Lebensdauer der Vorrichtung. Vorzugsweise ist die Vorrichtung frei von Permanentmagneten, so dass sie im abgeschalteten Zustand kein Magnetfeld erzeugt und unerwünschte Wirkungen so vermieden werden. Es ist bevorzugt, wenn die Vorrichtung abgesehen von den Haltekräften keine mechanischen Kräfte auf die Körperoberfläche und insbesondere auf die Schilddrüse ausübt, um so mechanische Reizungen zu vermeiden.

Das Magnetfeld erzeugende Modul ist erfindungsgemäß in einer Vorrichtung unter- bzw. eingebracht, die vorzugsweise so gestaltet ist, dass sie körpernah an dem Patienten befestigbar ist, so dass die Aktivierung der Schilddrüse möglichst optimal erfolgen kann. Der Umfang bzw. die Höhe der Aktivierung ist dabei z.B. über die Größe des Moduls, die Dauer der Applikation, die Stärke des Magnetfelds und/oder die Eigenschaften des Magnetfelds beeinflussbar.

Die Schilddrüse ist das Organ im menschlichen Körper, das über die Produktion von jodhaltigen Hormonen entscheidend die Stoffwechselaktivität steuert. Die Schilddrüse (lat. *Glandula thyr(e)oidea*) ist eine Hormondrüse, die beim Menschen am Hals unterhalb des Kehlkopfes vor der Luftröhre liegt. Beim Menschen hat sie die Form eines Schmetterlings.

Die Hauptfunktion der Schilddrüse besteht neben der Jodspeicherung in der Bildung der jodhaltigen Schilddrüsenhormone Thyroxin (Tetrajodthyronin, T₄) und Trijodthyronin (T₃). Die jodhaltigen Schilddrüsenhormone werden von den Follikelepithelzellen der Schilddrüse (Thyreozyten) gebildet und regulieren den Stoffwechsel und Funktionszustand fast aller Organe. Die Schilddrüsenhormone sind Bestandteil des sogenannten thyreotropen Regelkreises. Die Funktion der Schilddrüse wird hierbei durch den Hypothalamus und die Hirnanhangdrüse (Hypophyse) reguliert. In der Hirnanhangsdrüse wird das Hormon TSH (Thyreoidea stimulierendes Hormon) gebildet und in die Blutbahn abgegeben. An den Schilddrüsenzellen angelangt, fördert es deren Wachstum und die Ausschüttung von T₃ und T₄. Diese von der Schilddrüse ausgeschütteten Hormone wirken auf das Herz und den Kreislauf und führen zu einer Erhöhung der Herzfrequenz, des Blutdrucks und einer Erweiterung von Gefäßen. Zudem wirken sie auf den Zucker-, Fett- und Bindegewebsstoffwechsel, indem sie deren Umsatz steigern.

Durch die Wirkung der Schilddrüsenhormone werden folglich der Energieverbrauch und der Grundumsatz des Organismus reguliert. So ist es bekannt, dass eine Schilddrüsenunterfunktion (Hypothyreose) häufig zu Übergewicht und eine Schilddrüsenüberfunktion (Hyperthyreose) zu Untergewicht führen.

Den an sich bekannten Zusammenhang zwischen einer Schilddrüsenüberfunktion und dem damit oft einhergehenden Untergewicht des Patienten macht sich die Erfindung zu eigen, indem sie nämlich eine Aktivierung der Schilddrüse durch ein Magnetfeld bewirkt, und damit die Gewichtsreduktion induziert.

Beachtlich dabei ist, dass mit der erfindungsgemäßen Vorrichtung zwar über die Erhöhung/Steigerung der Schilddrüsentätigkeit das gewünschte Ziel der Gewichtsreduktion erreicht wird, dass aber die mit einer pathologischen Überfunktion der Schilddrüse oft einhergehenden problematischen Symptome, wie zum Beispiel Herzklopfen, Tremor oder Schlaflosigkeit im Wesentlichen ausbleiben. Die Verwendung eines Magnetfelds nach der Erfindung hat daher den besonderen Vorteil, eine eher moderate Aktivierung der Schilddrüse zu erreichen, die keine kritische Über-Aktivierung der Schilddrüse mit den dann ungewollten Folgen verursacht. Eine "Überdosierung" dieser Magnetfeldtherapie ist demnach *per se* ausgeschlossen. Nebenwirkungen können damit zumindest weitgehend vermieden werden. Im Sinne der Erfindung ist eine Aktivierung der Schilddrüse gleichbedeutend mit der Erhöhung oder Stimulation der Schilddrüsentätigkeit. Die Schilddrüsentätigkeit ist durch die Blutwerte des Patienten an freiem T3 (fT3) oder T4 (fT4) darstellbar. Sie ist außerdem erfassbar durch die Blutwerte an TSH.

Von einer Erhöhung der Aktivität der Schilddrüsentätigkeit ist immer dann auszugehen, wenn die fT3 und fT4 Werte eines Patienten über dessen individuellen Ausgangswert hinaus ansteigen. Bei einem Normwert über alle Menschen hinweg von 0,89 bis 1,70 ng/dl Blut (Serum) für fT4 und 2,0 bis 4,2 pg/ml Blut für fT3, liegt die individuelle Schwankungsbreite beim Menschen für fT4 und fT3 üblicherweise bei 1-2%. (Tageszeit- oder Jahreszeitabhängige Schwankungen dieser Hormone werden nicht beobachtet). Demnach gilt eine Erhöhung über 2% des jeweiligen individuellen Ausgangswerts als Aktivierung der Schilddrüse im Sinne dieser Erfindung.

Ebenso kann die Erniedrigung des individuellen TSH Blutwerts als Indikator für die Aktivierung der Schilddrüsentätigkeit herangezogen werden. Dieser Wert liegt beim Menschen im Allgemeinen zwischen 0,35 und 2,50 µlU/ml. Die individuelle Schwankung liegt auch hier bei ca. 1-2%. Erfindungsgemäß gilt eine Erniedrigung unter 2% des individuellen Ausgangswerts als - wenn auch mittelbares - Zeichen für eine erfindungsgemäße Aktivierung der Schilddrüsentätigkeit.

Diese Aktivierung der Schilddrüse durch die Verwendung der erfindungsgemäßen Vorrichtung ist insbesondere geeignet, Patienten mit einem Adipositas Grad I (Patienten mit einem BMI von 30 bis 34,9 kg/m²) oder Grad II (Patienten mit einem BMI von 35 bis 39,9 kg/m²)) zu behandeln. Es kann aber auch die Behandlung von Adipositas Grad III Patienten (BMI von mindestens 40) möglich sein. Insbesondere in diesen schweren Fällen ist die Kombination mit anderen therapeutischen Maßnahmen empfehlenswert.

Die erfindungsgemäße Vorrichtung hat den Vorteil, dass sie eine Gewichtsreduktion beim Adipositas-Patienten erreicht, ohne dass dieser seine Lebens-, Bewegungs- oder Essgewohnheiten umstellen oder verändern muss. Er kann daher sein gewohntes Leben fortsetzen und arbeitet gleichzeitig an dem von ihm aus medizinischen und/oder auch ästhetischen Gründen gewünschtem Ziel der Gewichtsabnahme.

Auch kann die Anwendung der erfindungsgemäßen Vorrichtung in nahezu jeder Lebenssituation erfolgen, so nämlich zum Beispiel während einer arbeitenden Tätigkeit, während Ruhephasen oder dem Sport, zu Hause oder auf Reisen. Dies ist vor allem in Hinblick auf eine chronische Anwendung wesentlich. Die erfindungsgemäße Vorrichtung führt demnach zu einer verbesserten Therapietreue der Patienten und ermöglichen eine bequeme Anwendung.

Die Vorrichtung besitzt außerdem den Vorteil, dass sie auch von medizinischen Laien anwendbar ist. So kann der Patient alleine, d.h. ohne Arzt oder eine medizinische Fachkraft die Vorrichtung an seinem Körper befestigen und das Generatormodul betätigen. Die erfindungsgemäße Vorrichtung erlaubt also eine einfache Anwendung.

Die Vorrichtung eignet sich nicht nur zur Behandlung von übergewichtigen Menschen, sondern auch von Tieren mit vergleichbaren Schwierigkeiten. So ist Übergewicht auch insbesondere bei Haustieren wie Hunden und Katzen ein Problem.

Die erfindungsgemäße Vorrichtung kann so gestaltet sein, dass sie körpernah getragen werden kann, d.h. entweder direkt am Körper des Patienten (Mensch oder Tier) oder aber an Kleidung oder Ausrüstung (Geschirr wie z.B. Halfter), die ihrerseits am Körper getragen wird. Das das Magnetfeld erzeugende Generatormodul sollte dabei vorzugsweise so in der Vorrichtung angebracht sein, dass es während der Verwendung der Vorrichtung auf die Schilddrüse, d.h. also auf den Hals des Patienten hin, ausrichtbar oder positionierbar sein kann.

Das Generatormodul kann an die Größe und/oder die Form der Schilddrüse angepasst sein. So kann das Generatormodul beispielsweise eine Schmetterlingsform aufweisen. In einer bevorzugten Ausführungsform beträgt die maximale Größe der dem Körper, insbesondere dem Hals anliegenden Fläche, höchstens 200 cm², bevorzugt höchstens 150 cm², insbesondere bevorzugt höchstens 100 cm² oder höchstens 50 cm².

In einer Ausführungsform ist die Vorrichtung als flächiger Träger, z.B. als Halsband, Halstuch, Schal oder Halskrause ausgestaltet. Dies ermöglicht eine große Kontaktfläche zwischen der Halsregion des Patienten und dem in der Vorrichtung angeordneten Generatormodul. Das Modul kann auf dem Träger aufliegen, oder aber in den Träger integriert sein. Ebenso ist eine Befestigung an oder eine Integration in halsnah getragenen Ausrüstungen (z.B. Kleidung) möglich, wie zum Beispiel in Jacken, Mänteln oder Westen.

Die Vorrichtung kann aber auch direkt auf der Haut befestigbar sein. In diesem Fall kann der Träger zum Beispiel als Pflaster ausgebildet sein.

Auch kann die Vorrichtung in Accessoires integriert sein, oder aber selber ein Accessoire bilden, beispielsweise ein Schmuckstück, Kopfband, Kragen, Schleier o.ä..

Die Befestigung der Vorrichtung am Körper kann in beliebig bekannten Weisen erfolgen. So ist die Ausgestaltung der Vorrichtung mit Befestigungselementen wie beispielsweise Druckknöpfen, Nieten, Knöpfen, Knebeln, Schnüren, Schnallen, Haken, Reißverschluss oder Klettverschluss denkbar.

Die erfindungsgemäße Vorrichtung kann als Textilerzeugnis vorliegen, nämlich beispielsweise im wesentlichen aus einem Gewebe, Gestricke, Gewirke, Filz- oder Walkware oder Flies gearbeitet sein. Vorteilhafterweise sind diese Textilien flexibel und passen sich somit an die zu behandelnde Körperregion möglichst optimal und individuell an. Vor allem in einer flächigen Ausgestaltung verbessern sie bei einer längeren Behandlungsdauer den Komfort und Annehmlichkeit des Patienten. Dies hat einen positiven Effekt auf die Therapietreue des Patienten.

In einer Ausführungsform kann die Vorrichtung selber ein Bekleidungsstück oder zumindest ein Teil davon sein. Dieses kann entsprechende Verstellelemente wie Klettverschlüsse, Knopfleisten o.ä. aufweisen, durch die die Vorrichtung an den einzelnen Patienten individuell angepasst werden kann.

Bei der Vorrichtung können die einzelnen Bestandteile in Hinblick auf ein möglichst geringes Gewicht ausgewählt werden, um den Tragekomfort zu erhöhen.

Die elektronischen Bestandteile der Vorrichtung, wie z.B. das Generatormodul, Kabel, oder die Steuerungselektronik, können so an der Vorrichtung befestigt sein, dass sie kein separates Bauteil darstellen, d.h. dass sie nicht zerstörungsfrei von der Vorrichtung lösbar sind. Dies kann für eines oder mehrere der elektronischen Bestandteile gelten.

Es ist aber auch denkbar, dass die elektronischen Bauteile von der Vorrichtung zerstörungsfrei trennbar sind. Dies kann vorteilhaft sein, um Bauteile auszutauschen oder zu reparieren.

Die elektronischen Bestandteile der Vorrichtung können auch entweder in Gänze oder in Teilen an Kleidung oder anderer Ausrüstung oder Accessoires des Patienten angebracht sein. In einer vorteilhaften Ausführungsform können die Bauteile für Dritte verdeckt angebracht sein (z.B. im Innenfutter oder der Innentasche einer Jacke, in einem Schal oder Halstuch, in einem Krawattenknoten oder einer Fliege) oder selber in Form eines Accessoires gestaltet sein.

Die Vorrichtung kann akustische, optische oder taktile (z.B. Vibrations-) Signale aussenden, die bestimmte Funktionszustände anzeigen (z.B. Ladezustand, Funktionsstörungen oder Ende der Behandlungsdauer) und/oder den Benutzer zu bestimmten Handlungen auffordern (z.B. An- oder Ablegen der Vorrichtung).

Zur Energieversorgung können Batterien, bevorzugt als Mignon A, Mignon AA oder Knopfzellen und diese auch als wieder aufladbare Batterien verwendet werden. Alternativ können auch andere stromerzeugende Vorrichtungen wie Solarzellen oder Miniatur-Windgeneratoren verwendet werden.

Das Generatormodul sendet (siehe oben) ein pulsierendes Magnetfeld aus.

In einer Ausführungsform weist das pulsierende Magnetfeld eine Frequenz von 1 Hz bis 100 KHz, bevorzugt 7 bis 20 Hz und besonders bevorzugt eine Frequenz von 14 Hz auf.

So hat es sich gezeigt, dass die Verwendung eines pulsierenden im Vergleich zu einem statischen Magnetfeld von großer Bedeutung für den Erfolg der erfindungsgemäßen Vorrichtung ist. Dabei lassen sich besonders gute Erfolge mit Frequenzen des pulsierenden Magnetfelds zwischen ca. 7 und 20 Hz und insbesondere ca. 14 Hz erzielen. Der erfindungsgemäße Erfolg kann auch bei Frequenzen erreicht werden, die sich von ca. 3 Hz bis ca. 60 Hz bewegen. Es ist z.B. erfindungsgemäß möglich, die Frequenz des pulsierenden Magnetfelds einer Schumann-Resonanzfrequenz anzupassen.

Somit ist eine Vorrichtung nach der Erfindung ganz besonders bevorzugt, die ein Generatormodul zur Erzeugung eines Magnetfeldes und eine Steuereinheit umfasst und körpernah am Säuger befestigbar ist und das Generatormodul in der Lage ist, ein pulsierendes Magnetfeld mit einer Frequenz von 1 Hz bis 100 KHz, bevorzugt 7 bis 20 Hz und besonders bevorzugt einer Frequenz von 14 Hz zu erzeugen.

Die Flussdichte des Magnetfelds kann von 0.1 bis 100 µT, bevorzugt von 20 bis 80 µT und besonders bevorzugt eine Flussdichte von 45 bis 55 µT betragen. Optional verändert sich die Flussdichte zeitabhängig, beispielsweise durch einen exponentiellen Anstieg und/oder Abfall.

In einer Ausführungsform der Erfindung ist die Impulsform des Magnetfeldes durch eine einfache Wellenfunktion darstellbar, besonders bevorzugt als Sinuskurve, als Rechteckkurve, als Trapezkurve oder als Sägezahnkurve. Optional ist die Impulsform (Schwingungsform) des Magnetfeldes zeitlich veränderlich, kann sich also beispielsweise im Lauf der Anwendung verändern.

Die Tragedauer der erfindungsgemäßen Vorrichtung ist individuell bestimmbar. Er kann sich über einen Zeitraum von wenigen Tagen, bis hin über Wochen und Monate erstrecken. Auch ist eine Daueranwendung möglich, die ggf. nur durch Schlafphasen des Patienten unterbrochen ist. Die tägliche Anwendung kann dabei ebenso individuell variiert werden. Sie kann einmal oder mehrfach täglich erfolgen. Ein Anwendungszyklus kann beispielsweise wenige Minuten bis aber auch einige Stunden betragen.

Es ist möglich, dass die Vorrichtung eine Steuerungseinheit umfasst, die eine individualisierbare Programmierung zur Erstellung eines individuellen Anwendungsplans erlaubt.

Die erfindungsgemäße Vorrichtung kann hinsichtlich der Eigenschaften der magnetischen Schwingungen in Bezug auf Stärke, Frequenz und/oder Impulsform und hinsichtlich gesteuert werden und ihr Einsatz bezüglich die Behandlungsdauer, Frequenz und Dauer der Einzelanwendung an die konkreten therapeutischen Notwendigkeiten angepasst werden. Auch diese Eigenschaften können in einer Ausführungsform der Erfindung über die Steuereinheit individualisiert und entsprechend programmiert werden.

Die Anwendung der erfindungsgemäßen Vorrichtung kann mit der Einnahme von Nahrungsergänzungsmitteln unterstützt werden. Bevorzugt sind polyphenolreiche Polyphenole und aromatische Verbindungen, die zwei oder mehr direkt an den aromatischen Ring gebundene Hydroxylgruppen enthalten und zu den sekundären Pflanzenstoffen gerechnet werden. Polyphenole sind pflanzliche Inhaltsstoffe, die insbesondere über eine antioxidative Wirkung die Gesundheit fördern. Beispiele für polyphenolreiche Lebensmittel sind Apfelbeeren, rote Weintrauben, Rotwein, die Mangostanfrucht (*Garcinia mangostana*), der Granatapfel (*Punica granatum*), Ginkgo, Tee, insbesondere grüner Tee, Zistrosen, die Samen von Perilla (*Perilla frutescen*), chinesische Zitronenmelisse, Kurkuma. Isolierte Polyphenole im Rahmen der Erfindung bezeichnen aus Pflanzen isolierte oder chemisch synthetisierte Polyphenole. Beispiele für isolierte Polyphenole sind Quercetin oder Resveratrol. Die erfindungsgemäße Vorrichtung und ihre Anwendung sind somit für eine Kombination mit den oben aufgeführten Stoffen geeignet.

In einer besonders bevorzugten Ausführungsform der Erfindung ist die Vorrichtung als Halsband so gestaltet, dass sie ein schaltbares Generatormodul zur Erzeugung eines Magnetfeldes, eine Steuerungselektronik und eine austauschbare Energieversorgung aufweist. Als Generatormodul werden in bevorzugter Weise eine Spule oder mehrere Spulen verwendet.

### Definitionen

Halsband meint im Rahmen der Erfindung einen flächigen Träger in band- oder schnurförmiger Ausgestaltung, der um den Hals gelegt werden kann..

Unter dem Begriff "Generatormodul" wird im Rahmen der Erfindung jegliche Vorrichtung verstanden, die in der Lage ist, ein statisches oder pulsierendes Magnetfeld zu erzeugen. Ein statisches Magnetfeld kann durch eines oder mehrere Permanentmagnete oder einen Elektromagneten ohne gepulsten Stromfluss erzeugt werden. In vorteilhafter Weise wird ein pulsierendes Magnetfeld eingesetzt, zu dessen Erzeugung das Generatormodul eine oder mehrere Spulen aus elektrisch leitendem Material (z.B. aus Kupferdraht) aufweist.

Der Begriff "pulsierendes Magnetfeld" im Rahmen der Erfindung bezeichnet allgemein ein Magnetfeld, das zeitlich veränderlich ist und dessen Feldstärke vorzugsweise im Zeitverlauf mehr als ein lokales Maximum aufweist. Beispielsweise sind exponentiell ansteigende und/oder exponentiell abfallende Impulse möglich. Vorzugsweise ist das pulsierende Magnetfeld ein sich zeitlich in periodischer Weise veränderndes Magnetfeld (auch als magnetische Schwingungen bezeichnet), das durch Frequenz, Impulsform und Feldstärke charakterisiert werden kann.

Der Begriff "Patient" kennzeichnet im Rahmen der Erfindung ein Säugetier, insbesondere einen Menschen.

Behandlung im Sinne der Erfindung bedeutet jede Maßnahme, die zur Vermeidung (Prävention oder Prophylaxe), Linderung oder Heilung eines Leidens bei Patienten beiträgt.

### Beispiel

Acht weibliche und zwei männliche Patienten mit deutlicher Schilddrüsenunterfunktion und leichter bis schwerer Adipositas - Grad 2-3 wurden mit der erfindungsgemäßen Vorrichtung über einen Zeitraum von vier Wochen behandelt, wobei das pulsierende Magnetfeld (Frequenz 14 Hz, Sinusschwingung, magnetische Flussdichte von 50uT) zweimal täglich für je 30 Minuten zur Anwendung kam. Die übrigen Lebensgewohnheiten der Patienten (d.h. Nahrungsaufnahme, Bewegung oder Schlafverhalten) blieben unverändert. Bei den Patienten wurde zur Beginn und zum Ende des Behandlungszeitraums die Blutwerte für das Schilddrüsen-stimulierende Hormon TSH (Thyreoidea-stimulierendes Hormon) und die beiden in freier Form vorliegenden Schilddrüsenhormone fT3 und fT4 bestimmt. Darüber hinaus wurden der Selenspiegel im Blut und das Gewicht der Patienten ermittelt.

### Ergebnisse

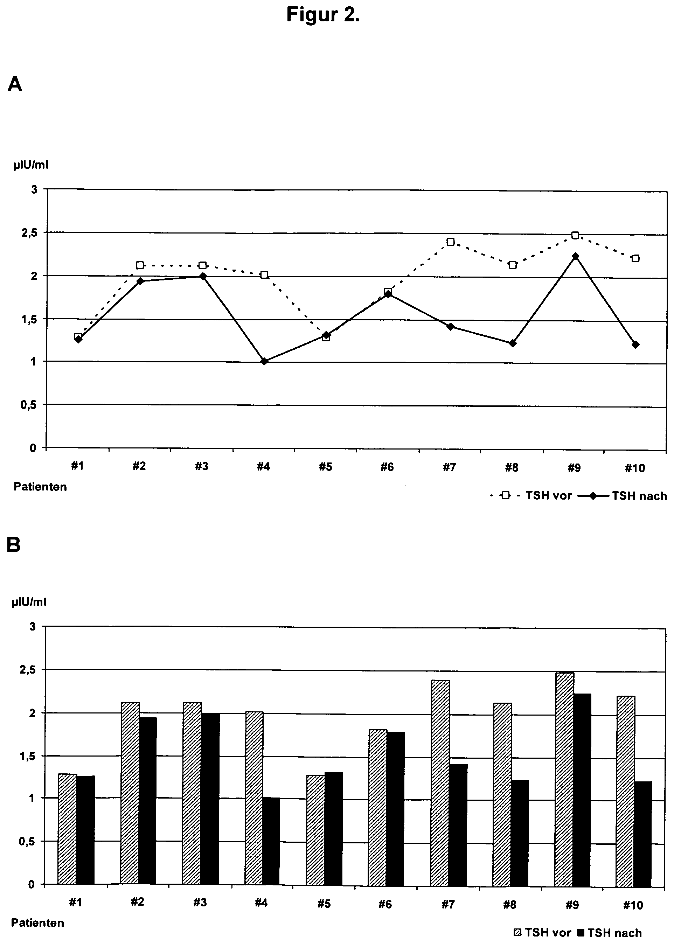

Bei der ganz überwiegenden Mehrzahl der Patienten zeigten sich Veränderungen im Gehalt von TSH, fT3 und fT4, wie sie für eine Aktivierung der Schilddrüse charakteristisch sind. Der Gehalt an TSH nimmt ab (s. Tabelle für Patienten No.1-3 und Figur 2 A und 2 B für die Patienten 4 bis 14), wobei die Ausschüttung des in der Hypophyse hergestellten TSH durch die Schilddrüsenhormone fT3 und fT4 gehemmt wird. Diese Hemmung erfolgt durch negatives Feedback im Rahmen des thyreotropen Regelkreises über die verringerte Produktion und Ausschüttung von TRH aus dem Hypothalamus.

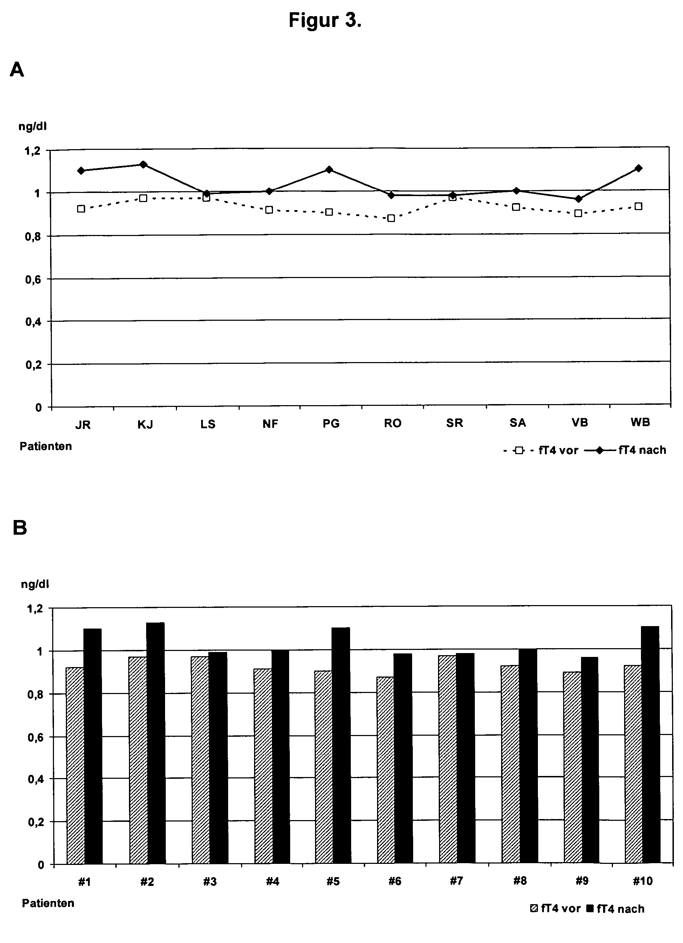

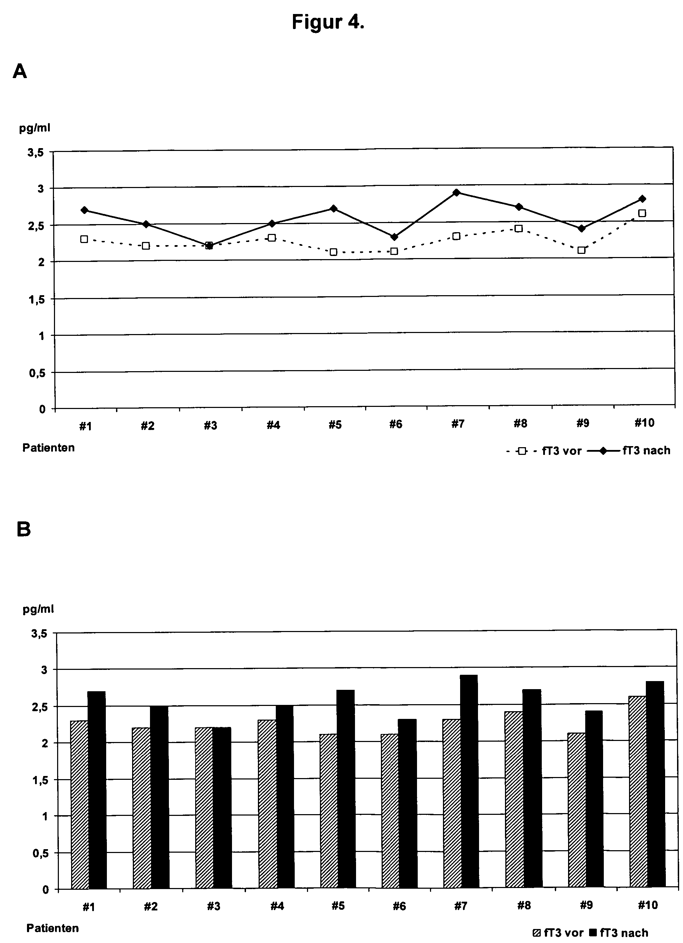

In erwartet komplementärer Weise war eine Zunahme der Plasmakonzentration an fT3 und fT4 in nahezu allen Patienten zu beobachten (s. Tabelle und Figuren 3 und 4). Die Konzentration an TSH nahm im gleichen Zeitraum ab.

Bei den Laboruntersuchungen wurde auch der Selen-Spiegel im Blut bestimmt. Selen spielt eine wichtige Rolle bei der Produktion der Schilddrüsenhormone, genauer bei der "Aktivierung" von Thyroxin (T4) zu Trijodthyronin (T3). Selen ist Bestandteil eines Enzyms, der Thyroxin-5'-Dejodase, die für die Entfernung eines Jodatoms aus T4 verantwortlich ist. Durch diese Dejodierung entsteht T3. Ein Selenmangel führt zu einem Mangel an Thyroxin-5'-Dejodase und dadurch zu einem T3-Mangel.

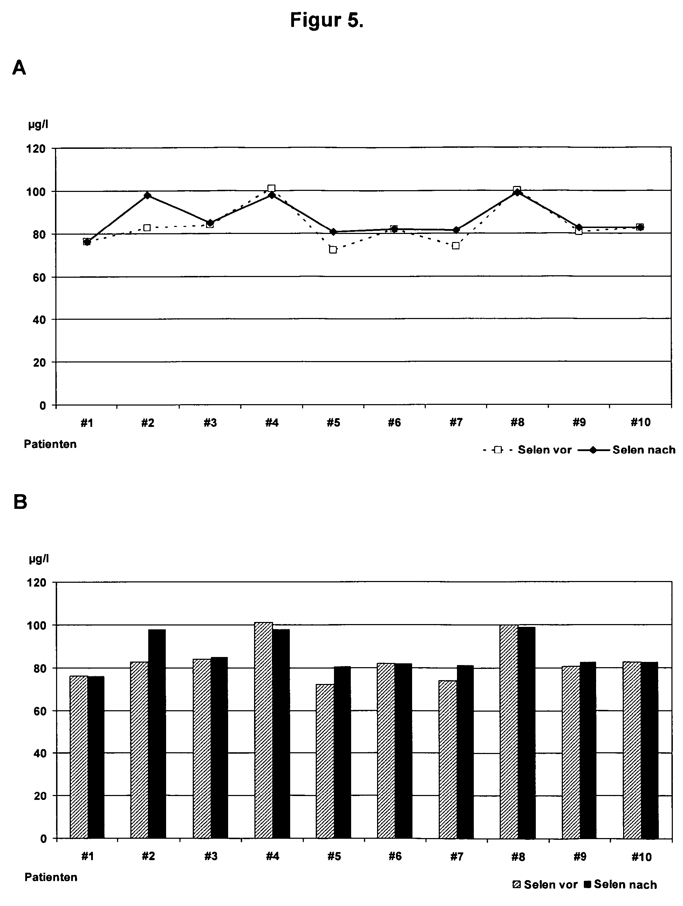

Durch die Behandlung mit einer erfindungsgemäßen Vorrichtung ("Thyreogym-Behandlung") wurde keine messbare Veränderung des Selen-Spiegels festgestellt (s. Figur 5).

**Tabelle 1: Tabellarische Zusammenstellung der gemessenen Plasmakonzentrationen an TSH (hypersensitiv), freiem Thyroxin (fT₄) und freiem Trijodthyronin (fT₃). Die Messung erfolgte vor ("vorher") und nach einem vierwöchigen Behandlungszeitraum mit der erfindungsgemäßen Vorrichtung ("nachher"). Die Behandlung erfolgte 2 mal täglich, jeweils für 30 min. Das Magnetfeld hatte 14 Hz.**

| Hormon | Patient #10 | | Patient #9 | | Patient #2 | |
|---|---|---|---|---|---|---|
| | | | | | | |

| | Vorher | nachher | vorher | nachher | vorher | Nachher |
|---|---|---|---|---|---|---|
| **TSH** (µlU/ml) | 2,22 | 1,22 (-45,0%) | 2,48 | 2,24 (-9,7%) | 2,12 | 1,94 (-8,5%) |
| **fT4** (ng/dl) | 0,92 | 1,1 (+ 19,6%) | 0,89 | 0,96 (+7,9%) | 0,97 | 1,13 (+16,5%) |
| **fT3** (ng/dl) | 2,6 | 2,8 (+7,7%) | 2,1 | 2,4 (+14,3%) | 2,2 | 2,5 (+13,6) |

Die Messung des Körpergewichtes zeigte bei den Patienten eine Gewichtsreduktion im Bereich von 4 bis 10 kg.

Nebenwirkungen wurden nicht beobachtet.

### Bewertung der Studienergebnisse

Das Verfahren führt somit eindeutig zu einer Aktivierung der Schilddrüse, wie sie durch die entsprechende Regulation der im thyreotropen Regelkreis essentiellen Hormone fT3, fT4 und TSH nachgewiesen kann. Die mit der Anwendung einhergehende Gewichtsreduktion kann somit durch die Aktivierung der Schilddrüse erklärt werden. Wichtig ist hierbei, dass der Anstieg der freien Schilddrüsenhormone in moderater Weise erfolgt ist, so dass die für eine medikamentöse Schilddrüsenaktivierung typischen Nebenwirkungen nicht beobachtet wurden.

### Vergleichsbeispiel

Drei weibliche Patienten mit leichter bis schwerer Adipositas - Grad 2-3 (Patienten 15 bis 17) wurden über einen Zeitraum von vier Wochen mit einem Verfahren behandelt, das dem oben angegebenen entsprach, bis auf die Tatsache, dass statt des pulsierenden Magnetfelds ein statisches Magnetfeld eingesetzt wurde. Die übrigen Lebensgewohnheiten der Patienten (d.h. Nahrungsaufnahme, Bewegung oder Schlafverhalten) blieben unverändert. Bei den Patienten wurde zur Beginn und zum Ende des Behandlungszeitraums die Blutwerte für TSH und fT3 und fT4 bestimmt.

| Hormon | Patient #15 | | Patient #16 | | Patient #17 | |
|---|---|---|---|---|---|---|
| | | | | | | |

| | vorher | nachher | vorher | nachher | vorher | nachher |
|---|---|---|---|---|---|---|
| **TSH** (µlU/ml) | 3,74 | 3,72 (-0,5%) | 10,47 | 10,50 (+0,3%) | 0,69 | 0,68 (-1,4%) |
| **fT4** (ng/dl) | 1,23 | 1,2 (-2,4%) | 1,30 | 1,30 (+/-0%) | 1,30 | 1,30 (+/-0%) |
| **fT3** (ng/dl) | 2,7 | 2,7 (+/-0%) | 2,6 | 2,8 (+7,7%) | 2,5 | 2,6 (+4,0) |

Das Vergleichsbeispiel zeigt, dass die Werte von TSH, fT4 und fT3 nicht signifikant verändert werden, wenn ein statisches Magnetfeld statt eines pulsierenden Magnetfelds verwendet wird. Auch die Körpergewichte der Patientinnen nach der Behandlung waren nicht signifikant anders als davor. Für die Erzielung des erfindungsgemäßen Erfolgs ist also die Verwendung eines pulsierenden Magnetfelds wesentlich.

Im Folgenden wird die Erfindung anhand eines Ausführungsbeispiels näher erläutert:

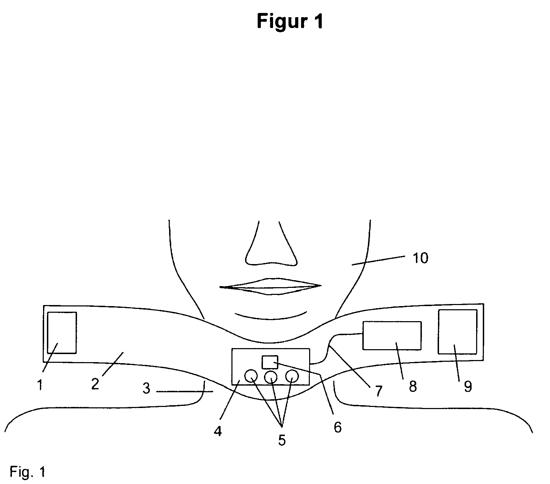

Die **Figur 1** zeigt eine erfindungsgemäße Vorrichtung in Form eines Halsbandes zur Anwendung zur Gewichtsreduktion.
   Die Vorrichtung zur Erzeugung von magnetischen Schwingungen ist in Form eines flexiblen, wattiert ausgefertigten Halsband **2** gestaltet. Dieses weist Befestigungselemente **1, 9** (z.B. einen Druckknopf oder ein Klettband) zum Schließen der Vorrichtung, eine elektronische Gerätesteuerung **4** (vorzugsweise in Form einer Platine, die zugänglich in einer Tasche angeordnet ist), eine oder mehrere Spulen **5** zur Erzeugung eines pulsierenden Magnetfeldes, ein Bedienelement **6** zur Steuerung der Elektronik, ein Verbindungskabel **7** zur Energieversorgung, und eine austauschbare Energieversorgung **8** (etwa eine austauschbare Batterie, die sich in einem wiederverschließbaren Fach befindet) auf.
   Der schematisch wiedergegebene Patient **10** veranschaulicht die Ausrichtung der Vorrichtung am Hals des Patienten. Zur Erhöhung des Tragekomforts ist im Halsband 2 eine Kinnmulde vorgesehen. Vorzugsweise sind an der Vorrichtung in einfach zugänglicher Lage Bedienelemente angeordnet (in der Figur nicht dargestellt), etwa einen Ein/Aus-Schalter oder gegebenenfalls Mittel zum Anpassen von Parametern des Magnetfelds (z.B. Regelung der Anschaltzeiten, -dauer und/oder -zyklen; Regelung der magnetischen Feldstärke bzw. Flussdichte; Regelung der Impulsform der magnetischen Schwingungen; Regelung der Frequenz der magnetischen Schwingungen).
**Figur 2** gibt die Konzentration an TSH im Blut der Patienten vor und nach der Thyreogymbehandlung wieder. Die Konzentration wurde in µg TSH /L Blut gemessen und ist in (**A**) als Liniendiagramm und in (**B**) als Balkendiagramm dargestellt.
**Figur 3** gibt die Konzentration an freiem T4 im Blut der Patienten vor und nach der Thyreogymbehandlung wieder. Die Konzentration wurde in ng fT4/dL Blut gemessen und ist in (**A**) als Liniendiagramm und in (**B**) als Balkendiagramm dargestellt.
**Figur 4** gibt die Konzentration an freiem T3 im Blut der Patienten vor und nach der Thyreogymbehandlung wieder. Die Konzentration wurde in pg fT3 /mL Blut gemessen und ist in (**A**) als Liniendiagramm und in (**B**) als Balkendiagramm dargestellt.
**Figur 5** gibt die Konzentration an Selen im Blut der Patienten vor und nach der Thyreogymbehandlung wieder. Die Konzentration wurde in µg Selen/L Blut gemessen und ist in (**A**) als Liniendiagramm und in (**B**) als Balkendiagramm dargestellt.

## Patentansprüche

1. Vorrichtung zur Behandlung von Übergewicht durch Aktivierung der Schilddrüse bei einem Säuger, die ein Generatormodul zur Erzeugung eines pulsierenden Magnetfeldes mit einer Frequenz von 3 Hz bis 60 Hz, bevorzugt einer Frequenz von 7 Hz bis 20 Hz und besonders bevorzugt einer Frequenz von 14 Hz und eine Steuereinheit umfasst , **dadurch gekennzeichnet, dass** sie am Hals des Säugers befestigbar ist.

2. Vorrichtung nach einem der vorherigen Ansprüche, **dadurch gekennzeichnet, dass** sie als flächiger Träger ausgestaltet ist.

3. Vorrichtung nach einem der vorherigen Ansprüche, **dadurch gekennzeichnet, dass** sie als Halsband ausgestaltet ist.

4. Vorrichtung nach einem der vorherigen Ansprüche, **dadurch gekennzeichnet, dass** die Steuerungseinheit eine Steuerung und/oder Programmierung des Magnetfelds bezüglich eines oder mehrerer der folgenden Parameter erlaubt:
a) An- und Ausschalten des Magnetfeldes
b) Regelung der Anschaltzeiten, -dauer und/oder -zyklen
c) Regelung der magnetischen Feldstärke oder Flussdichte
d) Regelung der Impulsform der magnetischen Schwingungen
e) Regelung der Frequenz der magnetischen Schwingungen

5. Vorrichtung nach einem der vorherigen Ansprüche, **dadurch gekennzeichnet, dass** das pulsierende Magnetfeld eine Feldstärke von 0,1 bis 100 µT, bevorzugt von 20 bis 80 µT und besonders bevorzugt von 45 bis 55 µT aufweist.

6. Vorrichtung nach einem der vorherigen Ansprüche, **dadurch gekennzeichnet, dass** die Impulsform des Magnetfeldes eine Sinuskurve, eine Rechteckkurve, eine Trapezkurve oder eine Sägezahnkurve darstellt.

## Claims

1. Device for treating obesity by activation of the thyroid gland in a mammal, which comprises a generator module for generating a pulsating magnetic field with a frequency of 3 Hz to 60 Hz, preferably a frequency of 7 Hz to 20 Hz, particularly preferably a frequency of 14 Hz, and a control unit, **characterized in that** it can be fastened to the neck of the mammal.

2. Device according to one of the preceding claims, **characterized in that** it is designed as a sheet-like support.

3. Device according to one of the preceding claims, **characterized in that** it is designed as a neck band.

4. Device according to one of the preceding claims, **characterized in that** the control unit allows the magnetic field to be controlled and/or programmed with respect to one or more of the following parameters:
a) switching the magnetic field on and off
b) controlling the switch-on times, durations and/or cycles
c) controlling the magnetic field strength or flux density
d) controlling the pulse shape of the magnetic oscillations
e) controlling the frequency of the magnetic oscillations.

5. Device according to one of the preceding claims, **characterized in that** the pulsating magnetic field has a field strength of 0.1 to 100 µT, preferably of 20 to 80 µT and particularly preferably of 45 to 55 µT.

6. Device according to one of the preceding claims, **characterized in that** the pulse shape of the magnetic field represents a sine curve, a rectangular curve, a trapezoidal curve or a saw tooth curve.

## Revendications

1. Dispositif de traitement de surpoids par activation de la thyroïde chez un mammifère, qui comprend un module de générateur pour générer un champ magnétique pulsé ayant une fréquence de 3 Hz à 60 Hz, de préférence une fréquence de 7 Hz à 20 Hz et particulièrement préférablement une fréquence de 14 Hz, et une unité de commande, **caractérisé en ce qu'**il peut être fixé à la gorge du mammifère.

2. Dispositif selon l'une quelconque des revendications précédentes, **caractérisé en ce qu'**il est configuré sous forme de support plat.

3. Dispositif selon l'une quelconque des revendications précédentes, **caractérisé en ce qu'**il est configuré sous forme de collier.

4. Dispositif selon l'une quelconque des revendications précédentes, **caractérisé en ce que** l'unité de commande permet une commande et/ou une programmation du champ magnétique par rapport à un ou plusieurs des paramètres suivants :
a) activation et désactivation du champ magnétique
b) régulation des temps, de la durée et/ou des cycles d'activation
c) régulation de l'intensité du champ magnétique ou de la densité du flux magnétique
d) régulation de la forme d'impulsion des oscillations magnétiques,
e) régulation de la fréquence des oscillations magnétiques.

5. Dispositif selon l'une quelconque des revendications précédentes, **caractérisé en ce que** le champ magnétique pulsant présente une intensité de champ de 0,1 à 100 µT, de préférence de 20 à 80 µT et particulièrement préférablement de 45 à 55 µT.

6. Dispositif selon l'une quelconque des revendications précédentes, **caractérisé en ce que** la forme d'impulsion du champ magnétique constitue une courbe sinusoïdale, une courbe rectangulaire, une courbe trapézoïdale ou une courbe en dent de scie.
